# EUROPEAN PATENT SPECIFICATION

(11) **EP 4 082 519 B1**
(45) Date of publication and mention of the grant of the patent: **25.12.2024**
(21) Application number: 21876726.7
(22) Date of filing: 09.11.2021
(51) Int. Cl.: A61K 8/73, A61K 8/60, A61K 31/715

(54) **DENDROBIUM OFFICINALE OLIGOSACCHARIDE, DENDROBIUM OFFICINALE OLIGOSACCHARIDE DERIVATIVE AND PREPARATION METHOD AND USE THEREOF**
DENDROBIUM-OFFICINALE-OLIGOSACCHARID, DENDROBIUM-OFFICINALE-OLIGOSACCHARID-DERIVAT SOWIE HERSTELLUNGSVERFAHREN DAFÜR UND VERWENDUNG DAVON
OLIGOSACCHARIDE DE DENDROBIUM OFFICINALE, DÉRIVÉ DE L'OLIGOSACCHARIDE DE DENDROBIUM OFFICINALE, SON PROCÉDÉ DE PRÉPARATION ET SON UTILISATION

(30) Priority: 05.03.2021 CN 202110247117
(43) Date of publication of application: 02.11.2022
(73) Proprietor: Kunming Institute of Botany, Chinese Academy of Sciences, Panlong District Kunming Yunnan 650201 (CN); Beijing Dr Plant Biotechnology Co., Ltd., Beijing 100055 (CN)
(72) Inventor: HU, Jiangmiao, Kunming, Yunnan 650201 (CN); YANG, Liu, Kunming, Yunnan 650201 (CN); WANG, Yuhua, Kunming, Yunnan 650201 (CN); HE, Rui, Kunming, Yunnan 650201 (CN); XIE, Yong, Kunming, Yunnan 650201 (CN)
(74) Representative: Canzler & Bergmeier Patentanwälte Partnerschaft mbB
(86) International application number: PCT/CN2021/129570
(87) International publication number: WO 2022/183763

(56) References cited:
- CN-A- 107 267 574
- CN-A- 108 635 300
- CN-A- 109 295 131
- CN-A- 109 535 269
- CN-A- 110 128 564
- CN-A- 113 004 432
- XING ZHANG ET AL: "Chemoenzymatic synthesis of unmodified heparin oligosaccharides: cleavage of p-nitrophenyl glucuronide by alkaline and Smith degradation", ORGANIC & BIOMOLECULAR CHEMISTRY, vol. 15, no. 5, 1 January 2017 (2017-01-01), pages 1222 - 1227, XP055753239, ISSN: 1477-0520, DOI: 10.1039/C6OB02603F
- HE TAO-BIN ET AL: "Structural characterization and immunomodulating activity of polysaccharide fromDendrobium officinale", INTERNATIONAL JOURNAL OF BIOLOGICAL MACROMOLECULES, ELSEVIER BV, NL, vol. 83, 1 December 2015 (2015-12-01), pages 34 - 41, XP029378138, ISSN: 0141-8130, DOI: 10.1016/J.IJBIOMAC.2015.11.038

## Description

### CROSS REFERENCE TO RELATED APPLICATION

This patent application claims the benefit and priority of Chinese Patent Application No. 202110247117.X filed on March 05, 2021, entitled "*Dendrobium officinale* oligosaccharide, *Dendrobium officinale* oligosaccharide derivative and preparation method and use thereof".

### TECHNICAL FIELD

The present disclosure relates to the technical field of natural medicine chemistry, and in particular to an oligosaccharide from *Dendrobium officinale,* a derivative of an oligosaccharide from *Dendrobium officinale* and a preparation method and use thereof.

### BACKGROUND ART

*Dendrobium officinale (Dendrobium officinale Kimura et Migo*) is a perennial herb of the genus *Dendrobium* in the family *Orchidaceae*, which is listed in each edition of the Pharmacopoeia. According to the records in the Pharmacopoeia, it has the effects of such as engendering liquid and nourishing stomach, nourishing yin and clearing heat, moistening lung and boosting kidney, and brightening eyes and strengthening lumbus. Modern pharmacological research shows that *Dendrobium officinale* has antioxidative, hypoglycemic, immunomodulatory and anti-inflammatory activities. It is recorded in ancient literatures that the stem of *Dendrobium officinale* can be used for moisturizing skin and treating diabetes. Studies have shown that *Dendrobium officinale* mainly contains polysaccharides, stilbene, flavonoids, phenanthrene, lignin and other chemical components.

Aging is an inevitable result of human growth, and the way to delay aging has always been the ultimate proposition in the fields of skin care products and biomedicine. Aging is roughly divided into two categories: physiological aging and pathological aging. Physiological aging refers to the physiological degeneration process that occurs after the mature period, and pathological aging is the senility changes caused by various external factors (including various diseases). The two are actually difficult to distinguish. It is recorded in ancient literatures that *Dendrobium officinale* has the anti-aging effects, and the natural, green, safe and efficient anti-aging substances in *Dendrobium officinale* are thus the focus of research.

At present, many domestic and foreign researchers have conducted research on the extraction and separation of chemical components in various parts of *Dendrobium officinale.* Most of the researchers mainly obtain small-molecule phenol components or polysaccharides with a molecular weight of more than 100,000. Some researchers have isolated 20 compounds from *Dendrobium officinale*, mainly bibenzil components, and have found that the small-molecule phenol components of *Dendrobium officinale* have antioxidative, anti-inflammatory and anti-tumor effects through activity screening. Some researchers have extracted and separated two polysaccharides from *Dendrobium officinale* with a molecular weight of 7.4×10⁵ Da and 5.4×10⁵ Da, respectively. Some researchers have used a method of water extraction, alcohol precipitation and sulfuric acid hydrolysis to obtain polysaccharides from *Dendrobium officinale*, and have found that the polysaccharides of *Dendrobium officinale* influence the count of peripheral white blood cells in mice through activity screening, indicating an immunomodulatory effect. Some researchers have obtained polysaccharides from *Dendrobium officinale* by ultrasonic extraction and have found that the polysaccharides of *Dendrobium officinale* influence the SOD, MDA and CAT of *Drosophila* through activity screening, indicating an antioxidative activity. Some researchers have demonstrated that there are mainly four homogeneous polysaccharides among polysaccharides of *Dendrobium officinale*, with a molecular weight in a range of 6,800-1.78×10⁵ Da. The previous research work mostly concentrates on the study of the pharmacological activity of the small-molecule phenol components and the polysaccharides, whereas there are few reports on the research of oligosaccharides.

CN 109295131 A discloses an oligosaccharide from *Dendrobium officinale* having seven glycosidic residues and a glucose residue at the non-reducing end.

CN 108635300 A discloses a polysaccharide from *Dendrobium officinale* having a small molecular.

Zhang et al. (Organic & Biomolecular Chemistry 15, 2017, 1222-1227) discloses a pnitrophenol derivative of a heptasaccharide having glucuronic acid at the reducing end.

### SUMMARY

In view of this, the present disclosure provides an oligosaccharide from *Dendrobium officinale*, a derivative of an oligosaccharide from *Dendrobium officinale* and a preparation method and use thereof. It is illustrated that the oligosaccharide from *Dendrobium officinale* has anti-aging and immunomodulatory effects, and is suitable for applying to skin cosmetics, health foods and medicines with anti-aging and immunomodulatory effects.

In order to achieve the above objectives, the present disclosure mainly provides the following technical solutions:
The present disclosure provides an oligosaccharide from *Dendrobium officinale*, having a structure represented by formula (I):

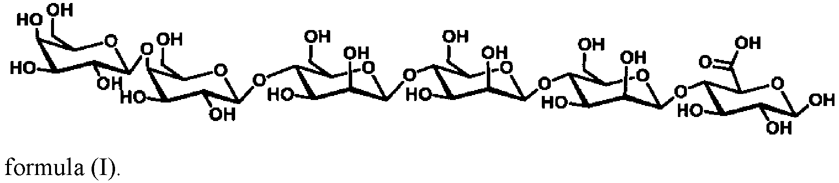

The present disclosure provides a derivative of an oligosaccharide from *Dendrobium officinale*, where the derivative is generated by reacting the glucuronic acid residue of the oligosaccharide from *Dendrobium officinale* according to the above technical solution with a hydrophobic residue, and the hydrophobic residue includes any one of linear or branched C₁-C₁₈ alkyl, C₁-C₁₈ alkyl carboxylic acid, C₁-C₁₈ alkyl amine, C₁-C₁₈ aryl alkyl and C₁-C₁₈ alkyl amide, or
the hydrophobic residue includes any one of benzoyl, phenylacetyl and phenylpropionyl.

The present disclosure provides the oligosaccharide from *Dendrobium officinale* or the derivative according to the above technical solutions for use in preparation of anti-aging or immunomodulatory products.

In some embodiments, the products include medicines, foods, formulations and cosmetics.

In some embodiments, a type of the cosmetics includes a face cream, an emulsion, a toning lotion or a repair cream.

The present disclosure provides a method for extracting the oligosaccharide from *Dendrobium officinale* described in above technical solutions, comprising:
1) preparation of a *Dendrobium officinale* powder: drying a stem of *Dendrobium officinale*, then smashing and sieving to obtain a *Dendrobium officinale* powder;
2) preparation of a water extract of *Dendrobium officinale:* mixing the *Dendrobium officinale* powder with water, then subjecting to a boiling water bath for one or more times, and then merging water extracts to obtain a water extract of *Dendrobium officinale*;
3) preparation of a concentrated water extract of *Dendrobium officinale:* centrifuging the water extract of *Dendrobium officinale*, and then concentrating under reduced pressure to obtain a concentrated water extract of *Dendrobium officinale*;
4) preparation of a redissolving liquid: adding ethanol to the concentrated water extract of *Dendrobium officinale*, precipitating, centrifuging and then discarding a supernatant to obtain a precipitate; adding water to the obtained precipitate for redissolving to obtain a redissolving liquid;
5) preparation of an aqueous solution of the oligosaccharide from *Dendrobium officinale*: deproteinizing the redissolving liquid, and freeze-drying to obtain a crude polysaccharide of *Dendrobium officinale*; adding water to dissolve the crude polysaccharide of *Dendrobium officinale*, then adding cellulase for a reaction, followed by inactivating and centrifuging, and discarding a precipitate to obtain an aqueous solution of the oligosaccharide from *Dendrobium officinale*;
6) preparation of the oligosaccharide from *Dendrobium officinale*: purifying the aqueous solution of the oligosaccharide from *Dendrobium officinale* to obtain the oligosaccharide from *Dendrobium officinale.*

In some embodiments, in step 1), a temperature for the drying is 50-60°C; a moisture mass content of the stem of *Dendrobium officinale* after the drying is less than 6%; a mesh number of the sieving is 20-40 meshes;
in step 2), the *Dendrobium officinale* powder and water are mixed in a mass-volume ratio of 1 : (50-80) g/mL; the boiling water bath is performed for 2-3 times; a time for each boiling water bath is 1-2 h;
in step 3), the centrifugation is conducted under conditions of 3000-4000 rpm/min, 8-12 min and room temperature; the concentration under reduced pressure is conducted under conditions of a rotary evaporation method, an ethanol recycling temperature of less than 50°C, and a pressure of 0.7 Pa-0.9 Pa; a material-liquid mass-volume ratio of the concentrated water extract of *Dendrobium officinale* is 1 : (5-10) g/mL.

In some embodiments, in step 4), the addition of ethanol to the concentrated water extract of *Dendrobium officinale* and the precipitation are specifically conducted as follows: adding ethanol with a volume concentration of 95% to the concentrated water extract of *Dendrobium officinale* until an alcohol concentration of a mixture is 70%-80% in volume percentage, and then precipitating overnight;
the centrifugation is conducted under conditions of 3500-4500 rpm, 18-22 min and room temperature; a weight-volume ratio of the precipitate and water for redissolving is 1 : (4-6) g/mL;
in step 5), the deproteinization of the redissolving liquid is conducted by a Sevage method;
a temperature for the freeze-drying is 45°C;
the addition of water to dissolve the crude polysaccharide of *Dendrobium officinale* is conducted as follows: adding water to the crude polysaccharide of *Dendrobium officinale* for dissolving until 4.5-5.5 mg/mL;
an added amount of the cellulase is 2%-4% of a weight of the crude polysaccharide of *Dendrobium officinale*;
after adding the cellulase, the reaction is conducted under a reaction temperature of 55-65°C for a reaction time of 2 h-4 h;
the inactivation is conducted by boiling for 25-35 min;
the centrifugation is conducted under conditions of 3500-4500 rpm, 15-25 min and room temperature;
an oligosaccharide content in the aqueous solution of oligosaccharide from *Dendrobium officinale* is greater than 80%;
in step 6), the purification is conducted by passing through a G75 gel column; a purity of the oligosaccharide from *Dendrobium officinale* is 99.86%.

The present disclosure provides a method for preparing the derivative described in the above technical solutions, comprising:
1) reducing a fatty acid into a fatty alcohol;
2) glycosylating the fatty alcohol with the oligosaccharide from *Dendrobium officinale* according to the above technical solutions to obtain the derivative.

In some embodiments, in step 1), a reaction process of the reduction of a fatty acid into a fatty alcohol is as follows: mixing 9-11 mmol of the fatty acid, 18-22 mmol of NaBH₄, 38-42 mmol of AlCl₃, 25-29 ml of THF, and 2-4 ml of MeOH for a reaction, and the reaction is conducted under a temperature of 95-105°C for a time of 7.5-8.5 h with a yield of 88-92%;
in step 2), a reaction process of the glycosylation is as follows: mixing the alcohol and the saccharide in a molar ratio of 1 : (8-10), and then using cetyl ammonium bromide as a catalyst for catalyzation, and the reaction is conducted under a temperature of 110-115°C for a time of 2.5-3.5 h with a yield of 78-82%.

Compared with the prior art, the oligosaccharide from *Dendrobium officinale,* the derivative and the preparation method and use thereof in the present disclosure have at least the following beneficial effects:

In one aspect, the present disclosure illustrates for the first time the oligosaccharides with a purity of more than or equal to 99% prepared from *Dendrobium officinale*, and carries out structural analysis to identify the structures of the above oligosaccharides from *Dendrobium officinale.*

Further, the present disclosure conducts for the first time a systematic study on the anti-aging activity of the above oligosaccharides from *Dendrobium officinale,* and has found that the above oligosaccharides from *Dendrobium officinale* can well inhibit collagenase and the like and thus have a strong anti-aging effect. Moreover, they also have a certain activity on TNF-α and IL-6, and a good immunomodulatory effect.

In another aspect, the preparation method and active application of the above oligosaccharides from *Dendrobium officinale* involved in the present disclosure provide a scientific basis for the application of *Dendrobium officinale* in related directions.

In yet another aspect, the oligosaccharide from *Dendrobium officinale* prepared by the present disclosure can be used as an intermediate for the development of *Dendrobium officinale* series products (such as application in the fields of medicine, food, daily necessities, etc.).

### BRIEF DESCRIPTION OF THE DRAWINGS

FIGs. 1-3 show the structure and purity identification HPLC-ELSD, NMR and MS spectra of the oligosaccharides from *Dendrobium officinale* of the present disclosure:
FIG. 1 shows the purity identification HPLC-ELSD spectrum of the oligosaccharides from *Dendrobium officinale*;
FIG. 2 shows the ¹H-NMR and ¹³C-NMR spectra of the oligosaccharides from *Dendrobium officinale*, where FIG. 2A is a ¹H-NMR spectrum, and FIG. 2B is a ¹³C-NMR spectrum;
FIGs. 3-6 show the test results of the anti-aging activity of the oligosaccharides from *Dendrobium officinale*:

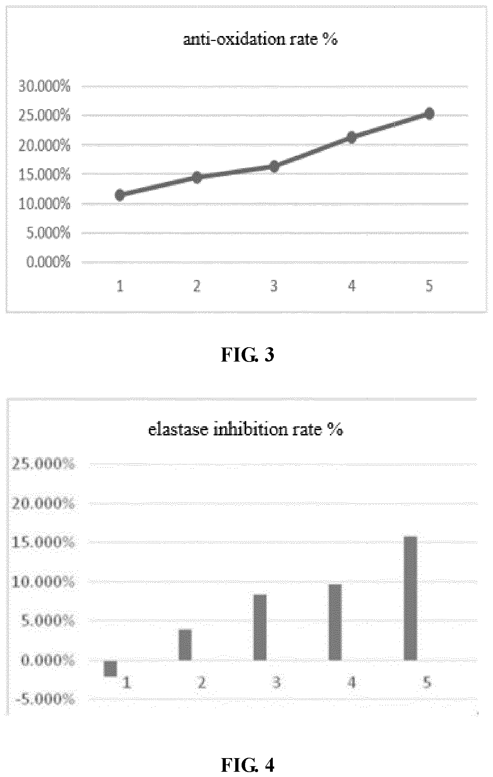

FIG. 3 shows the anti-oxidation rate of the oligosaccharides from *Dendrobium officinale* based on the scavenging rate of DPPH free radicals (oligosaccharide concentrations of 4%, 8%, 11%, 15%, 20%); where the ordinate is the anti-oxidation rate % represented by the scavenging rate of DPPH free radicals, and the abscissa (from 1 to 5) corresponds to the oligosaccharide concentration of 4%, 8%, 11%, 15%, 20%, respectively;
FIG. 4 shows the inhibition rate of the oligosaccharides from *Dendrobium officinale* on elastase activity (oligosaccharide concentrations of 4%, 8%, 11%, 15%, 20%); where the ordinate is the elastase inhibition rate %, and the abscissa (from 1 to 5) corresponds to the oligosaccharide concentration of 4%, 8%, 11%, 15%, 20%, respectively;

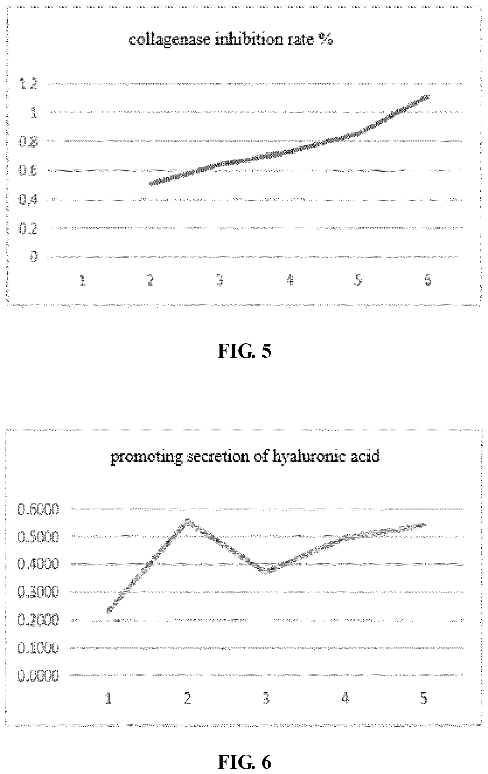

FIG. 5 shows the inhibition rate of oligosaccharides from *Dendrobium officinale* on collagenase activity (oligosaccharide concentrations of 0.4%, 0.8%, 1%, 2%, 4%); where the ordinate is the collagenase inhibition rate %, and the abscissa (from 1 to 5) corresponds to the oligosaccharide concentration of 0.4%, 0.8%, 1%, 2%, 4%, respectively;
FIG. 6 shows the activity of oligosaccharides from *Dendrobium officinale* on promoting the secretion of hyaluronic acid (oligosaccharide concentrations of 4%, 8%, 11%, 15%, 20%); where the ordinate is the secretion of hyaluronic acid (ng/mL), and the abscissa (from 1 to 5) corresponds to the oligosaccharide concentration of 4%, 8%, 11%, 15%, 20%, respectively;

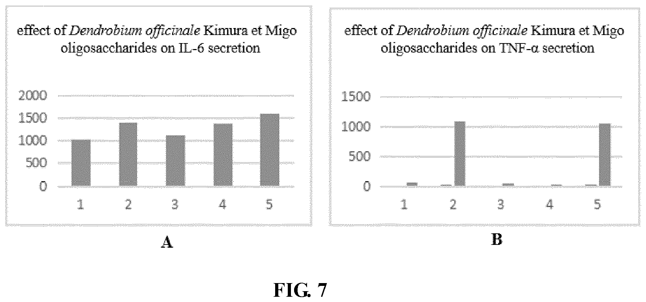

FIG. 7 shows the effect of oligosaccharides from *Dendrobium officinale* on the immunomodulatory activity of RAW264.7 cells (oligosaccharide concentrations of 3.125 µg/mL, 6.25 µg/mL, 12.5 µg/mL, 50 µg/mL, 100 µg/mL, 200 µg/mL); where A in FIG. 7 shows the effect of oligosaccharides on TNF-α secretion, the ordinate is TNF-α secretion pg/mL and the abscissa (from 1 to 5) corresponds to blank, lipopolysaccharide LPS and oligosaccharide with a final reaction concentration of 12.5 µg/mL, 6.25 µg/mL, 3.125 µg/mL, respectively; B in FIG. 7 shows the effect of oligosaccharides on IL-6 secretion, the ordinate is IL-6 secretion pg/mL, and the abscissa (from 1 to 5) corresponds to blank, lipopolysaccharide LPS and oligosaccharide with a final reaction concentration of 200 µg/mL, 100 µg/mL, 50 µg/mL, respectively.

### DETAILED DESCRIPTION OF THE EMBODIMENTS

In order to further explain the technical means and effects of the present disclosure to achieve the intended purpose of the present disclosure, the specific implementations, structures, features and effects of the disclosure according to the present disclosure will be described in detail below with reference to the accompanying drawings and preferred embodiments. In the following description, different "one embodiment" or "an embodiment" do not necessarily refer to the same embodiment. In addition, specific features, structures or characteristics in one or more embodiments may be combined in any suitable form.

The term "skin care product" in the present disclosure may refer to a skin care toner, a skin care lotion, a skin care mask, a skin cream, etc.; the skin care products in the embodiments of the present disclosure can also refer to cosmetics.

The present disclosure provides an oligosaccharide from *Dendrobium officinale*, having a structure represented by formula (I):

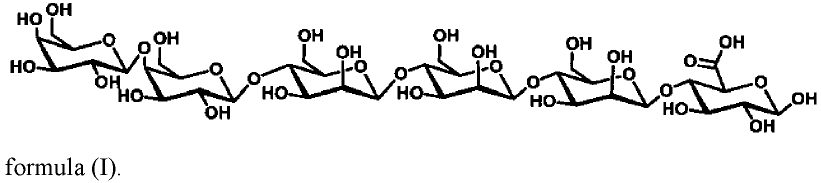

The present disclosure provides a method for extracting the oligosaccharide from *Dendrobium officinale* described in above technical solution, comprising:
1) preparation of *Dendrobium officinale* powder: drying a stem of Dendrobium officinale, then smashing and sieving to obtain the *Dendrobium officinale* powder;
   In some embodiments, a temperature for the drying is 50-60°C; a moisture mass content of the stem of *Dendrobium officinale* after the drying is less than 6%; and a mesh number of the sieving is 20-40 meshes;
2) preparation of a water extract of *Dendrobium officinale:* mixing the *Dendrobium officinale* powder with water, then subjecting to a boiling water bath for one or more times, and merging the water extracts obtained after the boiling water bath to obtain a water extract of *Dendrobium officinale*;
   in some embodiments, the *Dendrobium officinale* powder and water are mixed according to a mass-volume ratio of 1: (50-80) g/mL; the boiling water bath is performed for 2-3 times; and a time for each boiling water bath is 1-2 h;
3) preparation of a concentrated water extract of *Dendrobium officinale:* centrifuging the water extract of *Dendrobium officinale*, and then concentrating under reduced pressure to obtain a concentrated water extract of *Dendrobium officinale*;
   in some embodiments, the centrifugation is conducted under 3000-4000 rpm/min, 8-12 min, and room temperature; the concentration under reduced pressure is conducted by using a rotary evaporation method, an ethanol recycling temperature of less than 50°C and a pressure of 0.7 Pa-0.9 Pa; a material-liquid mass-volume ratio of the concentrated water extract of *Dendrobium officinale* is 1 : (5-10) g/mL;
4) preparation of a redissolving liquid: adding ethanol to the concentrated water extract of *Dendrobium officinale*, precipitating, centrifuging and then discarding a supernatant to obtain a precipitate; and then adding water to the obtained precipitate for redissolving to obtain a redissolving liquid;
   in some embodiments, ethanol with a volume concentration of 95% is added to the concentrated water extract of *Dendrobium officinale* until an alcohol concentration of the mixture is 70%-80% by volume percentage, and then the overnight precipitation is carried out; the conditions of centrifugation are 3500-4500 rpm, 18-22 min, and room temperature; and water is added according to a weight-volume ratio of the precipitate and water of 1: (4-6) g/mL for redissolving;
5) preparation of an aqueous solution of the oligosaccharide from *Dendrobium officinale*: deproteinizing the redissolving liquid, and freeze-drying to obtain a crude polysaccharide of *Dendrobium officinale*; and adding water to dissolve the crude polysaccharide of *Dendrobium officinale*, then adding cellulase for a reaction, followed by inactivating and centrifuging, and discarding a precipitate to obtain an aqueous solution of the oligosaccharide from *Dendrobium officinale*;
   in some embodiments, the redissolving liquid is deproteinized by a Sevage method; the freeze-drying temperature is 45°C; adding water to dissolve the crude polysaccharide of *Dendrobium officinale* to 4.5-5.5 mg/mL; an added amount of the cellulase is 2%-4% of the weight of the crude polysaccharide of *Dendrobium officinale*; after the cellulase is added, the reaction is conducted under a reaction temperature of 55-65°C, and a reaction time of 2 h-4 h; inactivating by boiling for 25-35 min; the centrifugation are conducted under 3500-4500 rpm, 15-25 min, and room temperature; the oligosaccharide content in the aqueous solution of the oligosaccharide from *Dendrobium officinale* is greater than 80%;
6) preparation of the oligosaccharide from *Dendrobium officinale*: purifying the aqueous solution of the oligosaccharide from *Dendrobium officinale* to obtain the oligosaccharide from *Dendrobium officinale*;
   in some embodiments, the purification is conducted by using a G75 gel column; the purity of the oligosaccharide from *Dendrobium officinale* is 99.86%.

The present disclosure provides the oligosaccharide from *Dendrobium officinale* described in above technical solutions for use in the preparation of medicines and formulations for anti-aging or immunomodulatory.

The present disclosure provides the oligosaccharide from *Dendrobium officinale* described in above technical solutions for use in the preparation of foods and cosmetics for anti-aging or immunomodulatory. In some embodiments, the cosmetics include a face cream, an emulsion, a toning lotion or a repair cream.

The present disclosure provides a derivative of an oligosaccharide from *Dendrobium officinale*, where the derivative is generated by reacting the glucose residue of the oligosaccharide from *Dendrobium officinale* according to the above technical solutions with a hydrophobic residue, and the hydrophobic residue includes any one of linear or branched C₁-C₁₈ alkyl, C₁-C₁₈ alkyl carboxylic acid, C₁-C₁₈ alkyl amine, C₁-C₁₈ aryl alkyl and C₁-C₁₈ alkyl amide, or
the hydrophobic residue includes any one of benzoyl, phenylacetyl, and phenylpropionyl (Replacing hydroxy and methoxy with phenyl).

The present disclosure provides a method for preparing the oligosaccharide derivative described in the above technical solutions, comprising:
1) reducing a fatty acid into a fatty alcohol;
   in some embodiments, a reaction process of reducing the fatty acid into the fatty alcohol is as follows: mixing 10 mmol of fatty acid, 20 mmol of NaBH₄, 40 mmol of AlCl₃, 27 ml of THF, 3 ml of MeOH for a reaction, the reaction temperature is 100°C, the reaction time is 8 h, and the yield is 90%;
2) glycosylating the fatty alcohol with the oligosaccharide from *Dendrobium officinale* according to the above technical solutions;
   in some embodiments, performing the glycosylation reaction of fatty alcohols obtained by reduction and the oligosaccharides from *Dendrobium officinale,* the glycosylation reaction is conducted as follows: a molar ratio of alcohol to saccharide is 1: (8-10), the catalyst is cetylammonium bromide, the reaction temperature is 110-115°C, the reaction time is 3 h, and the yield is 80%.

The present disclosure provides the derivative described in above technical solutions for use in the preparation of anti-aging or immunomodulatory products.

In some embodiments, the products include medicines, foods, formulations and cosmetics; in some embodiments, the cosmetics include a face cream, an emulsion, a toning lotion or a repair cream.

The present disclosure provides a method for separating and purifying oligosaccharides from *Dendrobium officinale.* The polysaccharide of *Dendrobium officinale* has a large molecular weight (more than or equal to 100,000) and is difficult to be absorbed through skin cells, and thus mainly acts on the outside of the skin and has a moisturizing effect, while the oligosaccharide formed by enzymatic hydrolysis of polysaccharides has a small molecular weight and is easily absorbed through skin cells, which plays an anti-aging effect. According to the different molecular weights of the two, they can be used differently.

On the other hand, it is also found that the oligosaccharides from *Dendrobium officinale* can increase the survival rate and survival time of Hacat and RAW264.7 cells. Considering that the oligosaccharides from *Dendrobium officinale* may have good anti-aging activities and immunomodulatory activities, the activities on several main target proteins (elastase, collagenase, hyaluronic acid) contributing to aging have been verified. The results show that the oligosaccharides from *Dendrobium officinale* have a strong inhibitory effect on collagenase and can effectively alleviate the degradation and metabolism of collagen, thus maintaining the youth state of skin. They also have a certain inhibitory effect on elastase, and meanwhile a certain activity to promote the secretion of hyaluronic acid. The oligosaccharides from *Dendrobium officinale* also have a certain activity on TNF-α and IL-6, and have a good immunomodulatory effect.

Hereinafter, the present disclosure is further described in detail with reference to the specific examples as follows.

### EXAMPLE 1

In this example, substantially, *Dendrobium officinale* oligosaccharides were separated and purified from a stem of *Dendrobium officinale*, and further subjected to structural identification:

The fresh stem of *Dendrobium officinale* was cut and placed in a blast drying box to dry at 55°C, and the moisture content was measured. When the moisture content reached 6% or less, the stem was smashed and passed through a 20-40 mesh sieve; 50 g of a *Dendrobium officinale* powder was taken, 2500 mL of ultrapure water was added thereto, then the mixture was subjected to boiling water bath for 2 times with 2 h each time, and the water extracts were merged. Then a centrifugation was conducted at 3000-4000 rpm for 10 min at room temperature of 25°C to obtain a water extract of *Dendrobium officinale*; the water extract of *Dendrobium officinale* was concentrated under reduced pressure to 500 mL to obtain a concentrated water extract of *Dendrobium officinale.* An appropriate amount of 95% ethanol was added to the concentrated water extract of *Dendrobium officinale* until an alcohol concentration of the mixture was 70-80%, then the mixture was precipitated overnight, centrifuged at 4000 rpm for 20 min at room temperature of 25°C, and the supernatant was discarded to obtain a precipitate; water was added according to the ratio of the weight of the precipitate to the volume of water of 1 : 5 (m : v) (g/mL) for redissolving to obtain a redissolving liquid. The redissolving liquid was deproteinized by the Sevage method, and then freeze-dried to obtain a crude polysaccharide of *Dendrobium officinale.* 200 mg of the crude polysaccharide of *Dendrobium officinale* was taken and dissolved with water to 5 mg/mL, cellulase was added thereto in 2%-4% of the weight of the polysaccharide of *Dendrobium officinale* and mixed evenly, then the mixture was reacted at 60°C for 2 h-4 h, boiled for 30 min for inactivation, centrifuged at 4000 rpm for 20 min at room temperature of 25°C, and a precipitate was discarded to obtain an aqueous solution of *Dendrobium officinale* oligosaccharide (the content of oligosaccharides in the aqueous solution of *Dendrobium officinale* oligosaccharide is greater than 80%). An appropriate amount of the aqueous solution of *Dendrobium officinale* oligosaccharide was taken and passed through a G75 gel column to obtain 150 mg of purified *Dendrobium officinale* oligosaccharide (with a purity of 99.86% and a molecular weight of about 1,000).

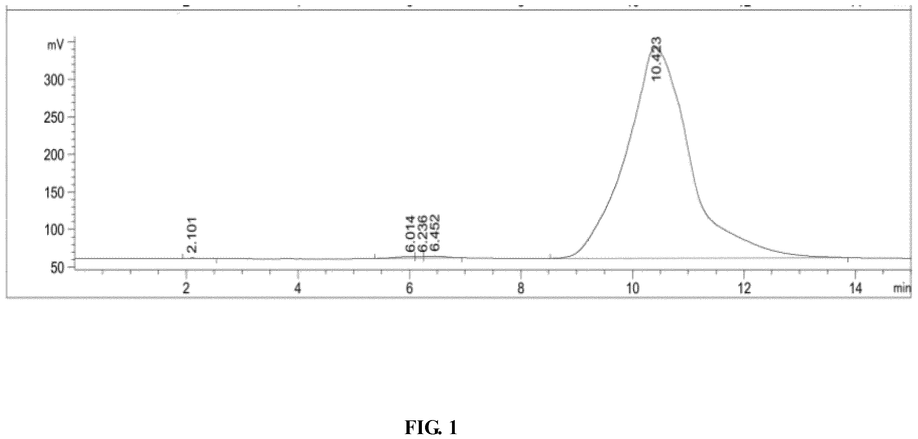

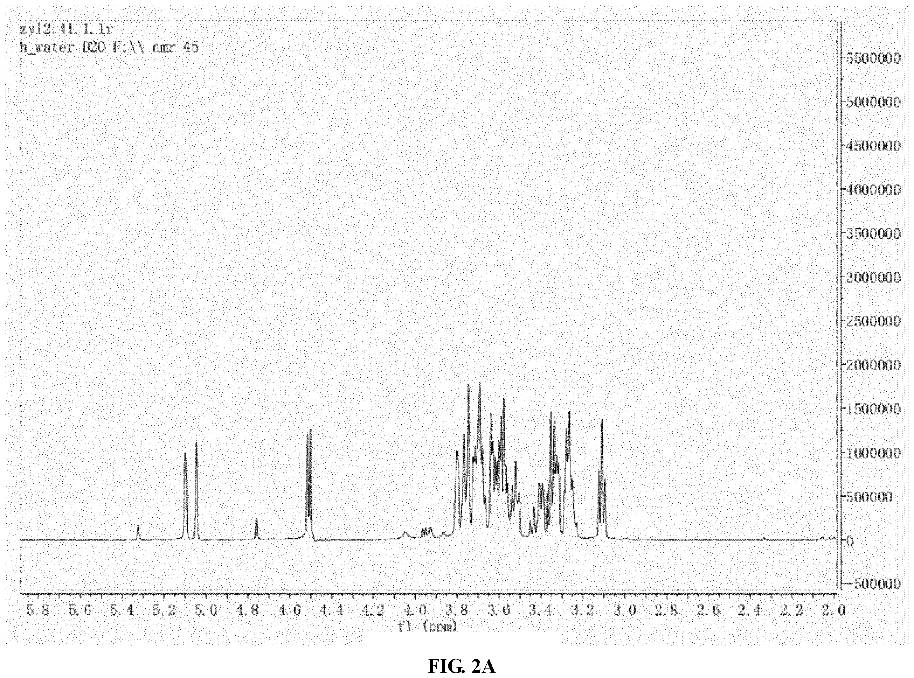

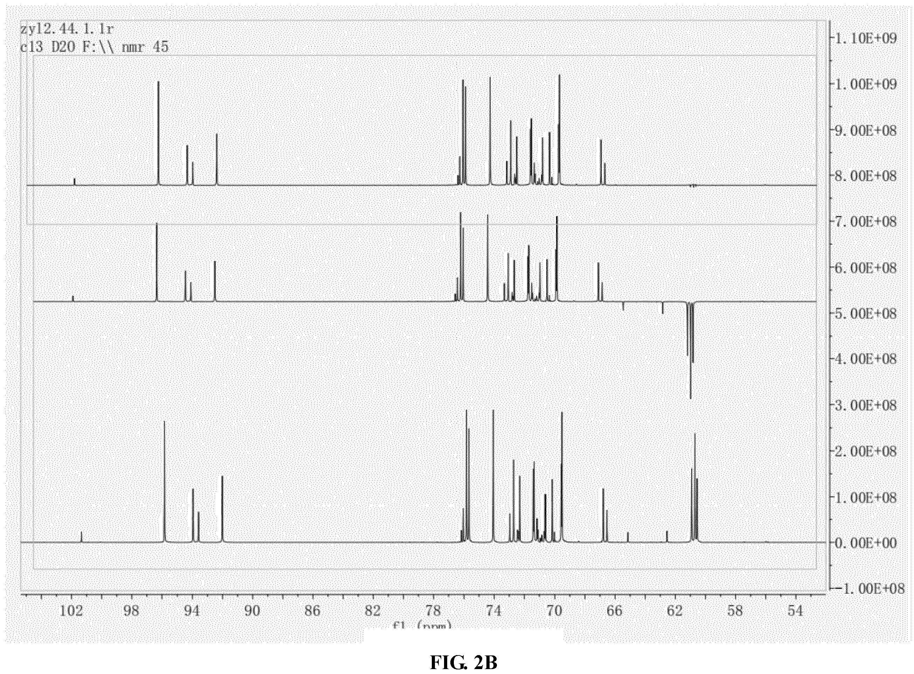

The purity of the *Dendrobium officinale* oligosaccharides was identified by high performance liquid chromatography, and the results are shown in FIG. 1. The structure of *Dendrobium officinale* oligosaccharides was identified by hydrogen nuclear magnetic resonance analysis and C13 nuclear magnetic resonance analysis, and the results are shown in FIG. 2.

### EXAMPLE 2

### Experiment on the DPPH free radical scavenging

### 1. Reagents:

DPPH, water-soluble vitamin E (Trolox) (Trolox dissolved in absolute ethanol, with a concentration of 10 mM, stored at -20°C), pure water.

### 2. Experimental method:

The drug to be tested was mixed with DPPH (a final concentration of 100 M) and reacted at 30°C for 1 h. 3 repeating wells were set, and a blank control well without drug and a Trolox positive control well were set at the same time. The OD value was measured by a microplate reader with a detection wavelength of 515 nm, and the anti-oxidation rate was calculated. Anti-oxidation rate (%) = (1-experimental well OD515mm/blank well OD515mm)×100%.

### 3. Experimental results:

As shown in Table 1.

**Table 1**

| Sample | Final reaction concentration | Anti-oxidation rate (%) |
|---|---|---|
| Trolox | 25 | 95.668% |
| *Dendrobium officinale* oligosaccharide | 4% | 11.335% |
| *Dendrobium officinale* oligosaccharide | 8% | 14.346% |
| *Dendrobium officinale* oligosaccharide | 11% | 16.213% |
| *Dendrobium officinale* oligosaccharide | 15% | 21.155% |
| *Dendrobium officinale* oligosaccharide | 20% | 25.217% |

### 4. Conclusion:

According to the data in Table 1, FIG. 3 is made for a clearer comparison. As can be seen from FIG. 3 and Table 1, the scavenging ability of DPPH free radicals of *Dendrobium officinale* oligosaccharides is evaluated in the experiment. The results show that the *Dendrobium officinale* oligosaccharides have a certain anti-oxidation rate (> 20%) at a concentration of 15%-20%.

### EXAMPLE 3

### Experiment on the elastase inhibition rate

### 1. Reagents:

Sample, positive control of catechin, Tris, HCl, porcine pancreatic elastase, N-succinyl-alanine-alanine-alanine-p-nitroaniline, DMSO.

### 2. Experimental method:

(1) Preparation of solution
   Preparation of a sample solution: 1 mg/mL sample solution was prepared (pure water or a buffer solution was used for preparation, and if insoluble in water or the buffer solution, the sample was firstly dissolved with DMSO, and then water or the buffer solution was added. In principle, the content of DMSO should not exceed 5%).
   Preparation of a positive control: 1 mg/mL catechin;
   Preparation of a buffer solution: 0.1 M of Tris-HCl buffer solution, PH = 8.0;
   Preparation of a reaction substrate solution:
      N-succinyl-alanine-alanine-alanine-p-nitroaniline (AAAPVN) solution (2 mM);
   Preparation: 28.5 µL of porcine pancreatic elastase solution (0.171 U/mL) was taken and dissolved in 10 mL of Tris-HCl buffer solution.
(2) The solution of 50 µL of sample, 50 µL of porcine pancreatic elastase and 100 µL of reaction substrate was added to a 96-well plate.
(3) After keeping at room temperature for 5 min, an absorbance at 420 nm was measured. Elastase inhibition rate (%) = (1-sample OD420mm/experimental well OD420mm) × 100%.

### 3. Experimental results:

As shown in Table 2.

**Table 2**

| Sample | Final reaction concentration | Elastase inhibition rate % |
|---|---|---|
| epicatechin gallate | 100 µg/ml | 68.540% |
| *Dendrobium officinale* oligosaccharide | 4% | -2.135% |
| *Dendrobium officinale* oligosaccharide | 8% | 3.886% |
| *Dendrobium officinale* oligosaccharide | 11% | 8.417% |
| *Dendrobium officinale* oligosaccharide | 15% | 9.723% |
| *Dendrobium officinale* oligosaccharide | 20% | 15.827% |

### 4. Conclusion:

According to the data in Table 2, FIG. 4 is made for a clearer comparison. As can be seen from FIG. 4 and Table 2, the elastase inhibition rate of *Dendrobium officinale* oligosaccharides is evaluated in the experiment. The results show that the *Dendrobium officinale* oligosaccharides have a certain elastase inhibition activity (> 10%) at a concentration of 20%.

### EXAMPLE 4

### Experiment on collagenase inhibition rate

### 1. Reagents:

Protease buffer solution, protease, protease substrate, inhibitor (1,10-Phenanthroline). All reagents were stored at -20°C.

2. Experimental method:
(1) Solution preparation:
The collagenase reaction mixture is shown in Table 3.

**Table 3**

| Composition | Content (%) |
|---|---|
| Collagenase buffer solution | 60 |
| Collagenase substrate | 40 |

(2) The compositions were designed as follows:
a. Background blank well: 100 µL/well of protease buffer solution.
b. Sample well: 100 µL, prepared into different concentrations.
c. Positive control well: 10 µL of protease + 90 µL of protease buffer solution.
d. Inhibitor control well: 10 µL of protease + 2 µL of inhibitor + 88 µL of protease buffer solution as shown in Table 4.

**Table 4**

| Group | Sample well (µl) | Positive control well (µl) | Inhibitor control well (µl) | Background blank well (µl) |
|---|---|---|---|---|
| Sample to be tested | 1-90 | - | - | - |
| Protease | 10 | 10 | 10 | - |
| Inhibitor | - | - | 2 | - |
| Protease buffer solution | Diluted to 100 | 90 | 88 | 100 |

(3) After adding reagents according to the above groups, 100 µL of collagenase reaction mixture was added to each well, incubated in the dark, and then the OD value at 345 nm was measured.

The incubation time depended on the activity of the sample to be tested, and the low activity sample could be incubated for 1-3 h. Collagenase inhibition rate (%) = (ODbackground well-ODsample well)(ODbackground well- ODpositive control well)

### 3. Experimental results:

As shown in Table 5.

**Table 5**

| Sample | Final reaction concentration | Collagenase inhibition rate (%) |
|---|---|---|
| Positive inhibitor | 1% | 102.23% |
| *Dendrobium officinale* oligosaccharide | 0.4% | 50.32% |
| *Dendrobium officinale* oligosaccharide | 0.8% | 63.63% |
| *Dendrobium officinale* oligosaccharide | 1% | 72.20% |
| *Dendrobium officinale* oligosaccharide | 2% | 84.80% |
| *Dendrobium officinale* oligosaccharide | 4% | 110.54% |

### 4. Conclusion:

According to the data in Table 5, FIG. 5 is made for a clearer comparison. As can be seen from FIG. 5 and Table 5, the collagenase inhibition rate of *Dendrobium officinale* oligosaccharides is evaluated in the experiment. The results show that the *Dendrobium officinale* oligosaccharides have a strong collagenase inhibition activity (> 50%) at a concentration of 0.4%-4%.

### EXAMPLE 5

### Experiment on promoting secretion of hyaluronic acid

### 1. Reagents and cells:

Human immortalized cortical cells (Hacat cells), human hyaluronic acid Elisa kit, distilled water.

### 2. Experimental method:

A 96-well plate was inoculated with 100 mL of cell suspension per well, and a concentration of the suspension was 4×10⁵ cells/mL. After 24 h of incubation, the original culture medium was discarded, then the cells were washed with PBS for 1-2 times and the serum-free medium containing different concentrations of the drug to be tested was added thereto, and continued to incubate for 24 h. A blank control without drugs and a TGF-β positive control were set. Then the cells were lysed with ripa lysis buffer solution containing 1% PMSF, the supernatant after centrifugation was taken, and the secretion of hyaluronic acid was detected according to the method provided in the collagen ELISA kit. The OD value was measured by a microplate reader with a detection wavelength of 450 nm. The OD value was substituted into the regression equation of the standard curve to calculate the increase rate of the secretion of hyaluronic acid.

### 3. Experimental results:

As shown in Table 6.

**Table 6**

| Sample | Final reaction concentration | Secretion of hyaluronic acid (ng/mL) |
|---|---|---|
| Blank control | | 0.2508 |
| *Dendrobium officinale* oligosaccharide | 4% | 0.2294 |
| *Dendrobium officinale* oligosaccharide | 8% | 0.5530 |
| *Dendrobium officinale* oligosaccharide | 11% | 0.3691 |
| *Dendrobium officinale* oligosaccharide | 15% | 0.4930 |
| *Dendrobium officinale* oligosaccharide | 20% | 0.5392 |

### 4. Conclusion:

According to the data in Table 6, FIG. 6 is made for a clearer comparison. As can be seen from FIG. 6 and Table 6, the secretion-promoting ability of hyaluronic acid of *Dendrobium officinale* oligosaccharides is evaluated. The results show that the *Dendrobium officinale* oligosaccharides have a certain activity to promote the secretion of hyaluronic acid (> 0.3 ng/mL) at a concentration of 8%-20%.

### EXAMPLE 6

Evaluation of the immunomodulatory activity of *Dendrobium officinale* oligosaccharides

### 1. Experimental cells and reagents:

RAW264.7 cells, TNF-α kit, IL-6 kit, distilled water.

### 2. Experimental methods:

The logarithmic-growth RAW264.7 cells were taken and inoculated into 12-well plates after cell counting. The plate was placed in an incubator overnight, and then the cell supernatant was discarded. Different concentrations of *Dendrobium officinale* oligosaccharides, lipopolysaccharides and basal medium 1640 were added under aseptic operation, and the supernatant after 24 h of intervention was collected and stored at -20°C for later use. The changes in TNF-α and IL-6 secretion were detected according to the instructions of the ELISA kit. The OD value was measured by a microplate reader with a detection wavelength of 450 nm. The OD value was substituted into the regression equation of the standard curve to calculate the increase rate of TNF-α and IL-6 secretion.

### 3. Experimental results:

As shown in Table 7.

**Table 7**

| Sample | Final reaction concentration (µg/mL) | TNF-α pg/mL | Final reaction concentration (µg/mL) | IL-6 pg/mL |
|---|---|---|---|---|
| Blank control | | 64.71 | | 1013.72 |
| LPS | | 1085.13 | | 1406.19 |
| *Dendrobium officinale* oligosaccharide | 12.5 | 53.86 | 200 | 1117.33 |
| *Dendrobium officinale* oligosaccharide | 6.25 | 35.66 | 100 | 1376.42 |
| *Dendrobium officinale* oligosaccharide | 3.125 | 1062.29 | 50 | 1593.01 |

### 4. Conclusion:

According to the data in Table 7, FIG. 7 is made for a clearer comparison. As can be seen from FIG. 7 and Table 7, the effects of *Dendrobium officinale* oligosaccharide on the immunomodulatory activity of RAW264.7 cells is evaluated. The results show that the secretion of TNF-α by RAW264.7 macrophages is significantly increased at a concentration of 3.125 µg/mL, which is equivalent to the positive control LPS. The secretion of IL-6 by RAW264.7 macrophages can be significantly increased at a concentration of 50 µg/mL, which is equivalent to the positive control LPS.

### EXAMPLE 7: glycosylation of fatty acids

### 1. Experimental reagents:

*Dendrobium officinale* oligosaccharides, fatty acids, NaBH₄, MeOH, metal salts, AlCl₃, THF, catalysts, etc.

2. Experimental methods and results:
1) Reducing a C₆ fatty acid into a fatty alcohol: 10 mmol of fatty acid, 20 mmol of NaBH₄, 40 mmol of AlCl₃, 27 mL of THF and 3 mL of MeOH were reacted at a temperature of 100°C for a time of 8 h with a yield of 90%;
2) Glycosylation: the reduced fatty alcohol and the *Dendrobium officinale* oligosaccharide were subjected to a glycosylation reaction with a molar ratio of the alcohol and the saccharide of 1 : (8-10); the catalyst was cetyl ammonium bromide; the reaction was conducted under a temperature of 110-115°C for a time of 3 h with a yield of 80%.

### 3. Results discussion:

The reaction results show that *Dendrobium officinale* oligosaccharides with terminal glycosides can also undergo the glycosylation reaction with fatty acids.

### EXAMPLE 8

This example provides an anti-aging or immunomodulatory face cream, comprising components in mass percentage: 0.4-0.6% of *Dendrobium officinale* stock solution rich in oligosaccharides, 7.5-8.5% of stearic acid, 1.5-2.5% of C₁₆ alcohol, 1.5-2.5% of self-emulsifying monoglyceride, 1.5-2.5% of hydrogenated lanolin, 11-13% of liquid paraffin, 6-8% of glycerin, 1-2% of an emulsifier, 0.1-0.3% of a preservative, 0.1-0.3% of an essence, and the balance of water. The face cream of the above formula was prepared according to a conventional method for preparing cosmetics.

### EXAMPLE 9

This example provides an anti-aging or immunomodulatory emulsion, comprising components in mass percentage: 0.4-0.6% of *Dendrobium officinale* stock solution rich in oligosaccharides, 1.3-1.5% of stearic acid, 0.08-0.12% of cetyl alcohol, 1.7-1.9% of 2-ethyl alcohol cetyl stearate, 0.1-0.3% of isopropyl myristate, 0.9-1.1% of 2-hexyl-1-decanol, 7-8% of liquid paraffin, 2.5-3.5% of glycerol, 7-9% of propylene glycol, 0.08-0.12% of triethanolamine, 0.3-0.4% of a carboxyvinyl polymer, 1.5-2.5% of Arlacel 165, 0.1-0.3% of a preservative, 0.1-0.3% of an essence, and the balance of water; The emulsion of the above formula was prepared according to a conventional method for preparing cosmetics.

### EXAMPLE 10

This example provides an anti-aging or immunomodulatory toner, comprising components in mass percentage: 1-10% of *Dendrobium officinale* stock solution rich in oligosaccharides, 0.3-0.4% of sodium polyacrylate, 3.5-4.5% of glycerol, 2-3% of 1,3-butanediol, 0.4-0.6% of vitamin B₅, 0.4-0.6% of arbutin, 0.04-0.06% of EDTA-Na₂, and the balance of deionized water; the method for preparing the toning lotion is as follows: the sodium polyacrylate was dissolved in water, stirred to fully swelling, then the remaining ingredients were added, and continued to stirring; then the *Dendrobium officinale* stock solution rich in oligosaccharides was added thereto, the mixture was stirred, and then deionized water was added to a constant volume to obtain the toner.

A preferred solution is as follows: 0.35 g of sodium polyacrylate was dissolved in 70 mL of water, stirred to fully swelling, then the remaining ingredients were added, and continued to stirring; then 10% of the *Dendrobium officinale* stock solution rich in oligosaccharides obtained in Example 1 was added thereto, the mixture was stirred uniformly, and then deionized water was added to 100 mL and packed.

### EXAMPLE 11

This example provides an anti-aging or immunomodulatory repair cream, comprising components in mass percentage: 5-7% of glycerin, 1.4-1.6% of carbomer, 1.4-1.6% of triethanolamine, 5.5-6.5% of propylene glycol, 0.15-0.25% of ethyl p-hydrobenzoate, 9-11% of *Dendrobium officinale* stock solution rich in oligosaccharides, 0.4-0.6% of an essential oil, and the balance of deionized water.

The method for preparing the repair cream is as follows: the carbomer was taken in an appropriate amount of deionized water, the mixture was stirred uniformly, let stand overnight to fully swelling, then glycerin was added, and the pH value was adjusted with triethanolamine to increase the viscosity of the gel matrix to obtain a carbomer gel; the *Dendrobium officinale* stock solution rich in oligosaccharides, propylene glycol and deionized water were mixed evenly, then the carbomer gel, essential oil and ethyl p-hydrobenzoate were added thereto, the resulting mixture was stirred until uniform and delicate, distilled water was added to a sufficient amount, and the mixture was grinded evenly to obtain a repair cream.

A preferred solution is as follows: 6 g of glycerin, 1.5 g of carbomer, 1.5 g of triethanolamine, 6 g of propylene glycol, 0.2 g of ethyl p-hydrobenzoate, 10% of *Dendrobium officinale* stock solution rich in oligosaccharide obtained in Example 1, and an appropriate amount of deionized water were added to a total weight of 100 g. The preparation method is as follows: the carbomer was taken in an appropriate amount of deionized water, the mixture was stirred uniformly, let stand overnight to fully swelling, then glycerin was added, and the pH value was adjusted with triethanolamine to increase the viscosity of the gel matrix to obtain a carbomer gel; a prescription amount of tanshinone extract, propylene glycol and deionized water were mixed evenly, then the carbomer gel, 0.5% of an essential oil and ethyl p-hydrobenzoate were added thereto, the resulting mixture was stirred until uniform and delicate, distilled water was added to a sufficient amount, and the mixture was grinded evenly to obtain a repair cream.

The above are only preferred embodiments of the present disclosure, and are not intended to be used to limit the present disclosure in any form.

## Claims

1. An oligosaccharide from *Dendrobium officinale*, having a structure represented by formula (I):

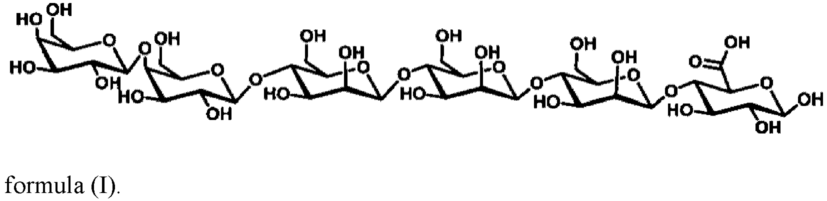

2. A derivative of an oligosaccharide from *Dendrobium officinale*, wherein the derivative is generated by reacting the glucuronic acid residue of the oligosaccharide from *Dendrobium officinale* according to claim 1 with a hydrophobic residue, and the hydrophobic residue comprises any one of linear or branched C₁-C₁₈ alkyl, C₁-C₁₈ alkyl carboxylic acid, C₁-C₁₈ alkyl amine, C₁-C₁₈ aryl alkyl and Ci-Cis alkyl amide, or
the hydrophobic residue comprises any one of benzoyl, phenylacetyl and phenylpropionyl.

3. The oligosaccharide from *Dendrobium officinale* according to claim 1 or the derivative according to claim 2 for use in preparation of anti-aging or immunomodulatory products.

4. The oligosaccharide from *Dendrobium officinale* or the derivative according to claim 3, wherein the products comprise medicines, foods, formulations and cosmetics.

5. The oligosaccharide from *Dendrobium officinale* or the derivative according to claim 4, wherein a type of the cosmetics comprises a face cream, an emulsion, a toning lotion or a repair cream.

6. A method for extracting the oligosaccharide from *Dendrobium officinale* according to claim 1, comprising:
1) preparation of a *Dendrobium officinale* powder: drying a stem of *Dendrobium officinale*, then smashing and sieving to obtain a *Dendrobium officinale* powder;
2) preparation of a water extract of *Dendrobium officinale:* mixing the *Dendrobium officinale* powder with water, subjecting to a boiling water bath for one or more times, and then merging water extracts to obtain a water extract of *Dendrobium officinale*;
3) preparation of a concentrated water extract of *Dendrobium officinale:* centrifuging the water extract of *Dendrobium officinale*, and then concentrating under reduced pressure to obtain a concentrated water extract of *Dendrobium officinale*;
4) preparation of a redissolving liquid: adding ethanol to the concentrated water extract of *Dendrobium officinale*, precipitating, centrifuging and then discarding a supernatant to obtain a precipitate; adding water to the obtained precipitate for redissolving to obtain a redissolving liquid;
5) preparation of an aqueous solution of the oligosaccharide from *Dendrobium officinale*: deproteinizing the redissolving liquid, and freeze-drying to obtain a crude polysaccharide of *Dendrobium officinale*; adding water to dissolve the crude polysaccharide of *Dendrobium officinale*, then adding cellulase for a reaction, followed by inactivating and centrifuging, and discarding a precipitate to obtain an aqueous solution of the oligosaccharide from *Dendrobium officinale*;
6) preparation of the oligosaccharide from *Dendrobium officinale*: purifying the aqueous solution of the oligosaccharide from *Dendrobium officinale* to obtain the oligosaccharide from *Dendrobium officinale.*

7. The method according to claim 6, wherein,
in step 1), a temperature for the drying is 50-60°C; a moisture mass content of the stem of *Dendrobium officinale* after the drying is less than 6%; a mesh number of the sieving is 20-40 meshes;
in step 2), the *Dendrobium officinale* powder and water are mixed in a mass-volume ratio of 1 : (50-80) g/mL; the boiling water bath is performed for 2-3 times; a time for each boiling water bath is 1-2 h;
in step 3), the centrifugation is conducted under conditions of 3000-4000 rpm/min, 8-12 min and room temperature; the concentration under reduced pressure is conducted under conditions of a rotary evaporation method, an ethanol recycling temperature of less than 50°C and a pressure of 0.7 Pa-0.9 Pa; a material-liquid mass-volume ratio of the concentrated water extract of *Dendrobium officinale* is 1 : (5-10) g/mL;
in step 4), the addition of ethanol to the concentrated water extract of *Dendrobium officinale* and the precipitation are specifically conducted as follows: adding ethanol with a volume concentration of 95% to the concentrated water extract of *Dendrobium officinale* until an alcohol concentration of a mixture is 70%-80% in volume percentage, and then precipitating overnight;
the centrifugation is conducted under conditions of 3500-4500 rpm, 18-22 min and room temperature; a weight-volume ratio of the precipitate and water for the redissolving is 1 : (4-6) g/mL;
in step 5), the deproteinization of the redissolving liquid is conducted by a Sevage method;
a temperature for the freeze-drying is 45°C;
the addition of water to dissolve the crude polysaccharide of *Dendrobium officinale* is conducted as follows: adding water to the crude polysaccharide of *Dendrobium officinale* for dissolving until 4.5-5.5 mg/mL;
an added amount of the cellulase is 2%-4% of a weight of the crude polysaccharide of *Dendrobium officinale*;
after adding the cellulase, the reaction is conducted under a reaction temperature of 55-65°C for a reaction time of 2 h-4 h;
the inactivation is conducted by boiling for 25-35 min;
the centrifugation is conducted under conditions of 3500-4500 rpm, 15-25 min and room temperature;
an oligosaccharide content in the aqueous solution of the oligosaccharide from *Dendrobium officinale* is greater than 80%;
in step 6), the purification is conducted by passing through a G75 gel column; a purity of the oligosaccharide from *Dendrobium officinale* is 99.86%.

8. A method for preparing the derivative according to claim 2, comprising:
1) reducing a fatty acid into a fatty alcohol;
2) glycosylating the fatty alcohol with the oligosaccharide from *Dendrobium officinale* according to claim 1 to obtain the derivative.

9. The method according to claim 8, wherein in step 1), a reaction process of the reduction of a fatty acid into a fatty alcohol is as follows: mixing 9-11 mmol of the fatty acid, 18-22 mmol of NaBH₄, 38-42 mmol of AlCl₃, 25-29 ml of THF and 2-4 ml of MeOH for a reaction, and the reaction is conducted under a temperature of 95-105°C for a time of 7.5-8.5 h with a yield of 88-92%;
in step 2), a reaction process of the glycosylation is as follows: mixing the alcohol and the saccharide in a molar ratio of 1 : (8-10), and then using cetyl ammonium bromide as a catalyst for catalyzation, and the reaction is conducted under a temperature of 110-115°C for a time of 2.5-3.5 h with a yield of 78-82%.

## Patentansprüche

1. Oligosaccharid aus Dendrobium *officinale* mit einer Struktur, die durch die Formel (I) dargestellt wird:

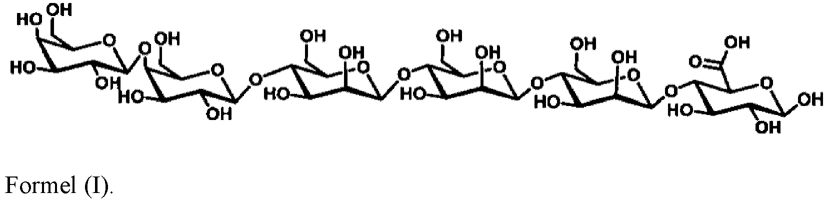

2. Derivat eines Oligosaccharids aus Dendrobium officinale, wobei das Derivat durch Reagieren des Glucuronsäurerests des Oligosaccharids aus Dendrobium officinale gemäß Anspruch 1 mit einem hydrophoben Rest erzeugt wird, und der hydrophobe Rest ein beliebiges von linearem oder verzweigtem C1-C18-Alkyl, C1-C18-Alkylcarbonsäure, C1-C18-Alkylamin, C1-C18-Arylalkyl und C1-C18-Alkylamid umfasst, oder
der hydrophobe Rest ein beliebiges von Benzoyl, Phenylacetyl und Phenylpropionyl umfasst.

3. Oligosaccharid aus Dendrobium *officinale* gemäß Anspruch 1 oder Derivat gemäß Anspruch 2 zur Verwendung bei der Herstellung von Anti-Aging- oder immunmodulatorischen Produkten.

4. Oligosaccharid aus Dendrobium *officinale* oder Derivat gemäß Anspruch 3, wobei die Produkte Arzneimittel, Nahrungsmittel, Rezepturen und Kosmetika umfassen.

5. Oligosaccharid aus Dendrobium *officinale* oder Derivat gemäß Anspruch 4, wobei eine Art der Kosmetika eine Gesichtscreme, eine Emulsion, eine Tönungslotion oder eine Reparaturcreme umfasst.

6. Verfahren zum Extrahieren des Oligosaccharids aus Dendrobium *officinale* gemäß Anspruch 1, umfassend:
1) Herstellung eines Dendrobium *officinale-Pulvers:* Trocknen eines Stamms von Dendrobium *officinale,* dann Zerkleinern und Sieben, um ein Dendrobium *officinale-Pulver* zu erhalten;
2) Herstellung eines Wasserextrakts von Dendrobium *officinale:* Mischen des Dendrobium *officinale-Pulvers* mit Wasser, ein- oder mehrmaliges Aussetzen einem siedenden Wasserbad und dann Vermischen von Wasserextrakten, um einen Wasserextrakt von Dendrobium *officinale* zu erhalten;
3) Herstellung eines konzentrierten Wasserextrakts von Dendrobium officinale: Zentrifugieren des Wasserextrakts von Dendrobium *officinale* und dann Konzentrieren unter reduziertem Druck, um einen konzentrierten Wasserextrakt von Dendrobium *officinale* zu erhalten;
4) Herstellung einer wiederauflösenden Flüssigkeit: Zugeben von Ethanol zu dem konzentrierten Wasserextrakt von Dendrobium *officinale,* Kondensieren, Zentrifugieren und dann Verwerfen eines Überstands, um ein Kondensat zu erhalten; Zugeben von Wasser zu dem erhaltenen Kondensat zum Wiederauflösen, um eine wiederauflösende Flüssigkeit zu erhalten;
5) Herstellung einer wässrigen Lösung des Oligosaccharids aus Dendrobium *officinale*: Deproteinisieren der wiederauflösenden Flüssigkeit und Gefriertrocknen, um ein Rohpolysaccharid von Dendrobium *officinale* zu erhalten; Zugeben von Wasser, um das Rohpolysaccharid von Dendrobium *officinale* aufzulösen, dann Zugeben von Cellulase für eine Reaktion, gefolgt von Inaktivieren und Zentrifugieren, und Verwerfen eines Kondensats, um eine wässrige Lösung des Oligosaccharids aus Dendrobium *officinale* zu erhalten;
6) Herstellung des Oligosaccharids aus Dendrobium officinale: Reinigen der wässrigen Lösung des Oligosaccharids aus Dendrobium officinale, um das Oligosaccharid aus Dendrobium *officinale* zu erhalten.

7. Verfahren gemäß Anspruch 6, wobei
in Schritt 1) beträgt eine Temperatur für das Trocknen 50-60 °C; ein Feuchtigkeitsmassengehalt des Stamms von Dendrobium officinale nach dem Trocknen beträgt weniger als 6 %; eine Maschenzahl des Siebens beträgt 20-40 Maschen;
in Schritt 2) werden das Dendrobium *officinale*-Pulver und Wasser in einem Masse-Volumen-Verhältnis von 1 : (50-80) g/ml gemischt; das siedende Wasserbad wird 2-3 Mal durchgeführt; eine Zeit für jedes siedende Wasserbad beträgt 1-2 h;
in Schritt 3) wird die Zentrifugation unter Bedingungen von 3000-4000 U/min, 8-12 min und Raumtemperatur durchgeführt; die Konzentration unter reduziertem Druck wird unter Bedingungen eines Rotationsverdampfungsverfahrens, einer Ethanol-Recyclingtemperatur von weniger als 50 °C und einem Druck von 0,7 Pa-0,9 Pa durchgeführt; ein Masse-Volumen-Verhältnis von Material zu Flüssigkeit des konzentrierten Wasserextrakts von Dendrobium *officinale* beträgt 1 : (5-10) g/ml;
in Schritt 4) werden das Zugeben von Ethanol zu dem konzentrierten Wasserextrakt von Dendrobium *officinale* und die Kondensation spezifisch wie folgt durchgeführt: Zugeben von Ethanol mit einer Volumenkonzentration von 95 % zu dem konzentrierten Wasserextrakt von Dendrobium *officinale,* bis eine Alkoholkonzentration einer Mischung 70 %-80 % in Volumenprozent beträgt, und dann Kondensieren über Nacht;
die Zentrifugierung wird unter Bedingungen von 3500-4500 U/min, 18-22 min und Raumtemperatur durchgeführt; ein Gewicht-Volumen-Verhältnis des Kondensats und Wasser für das Wiederauflösen beträgt 1 : (4-6) g/ml;
in Schritt 5) wird die Deproteinisierung der wiederauflösenden Flüssigkeit durch ein Sevage-Verfahren durchgeführt;
eine Temperatur für das Gefriertrocknen 45 °C beträgt;
die Zugabe von Wasser, um das Rohpolysaccharid von Dendrobium *officinale* aufzulösen, wird wie folgt durchgeführt: Zugeben von Wasser zu dem Rohpolysaccharid von Dendrobium *officinale* zum Auflösen bis 4,5-5,5 mg/ml;
eine zugegebene Menge der Cellulase beträgt 2 %-4 % eines Gewichts des Rohpolysaccharids von Dendrobium *officinale*,
nach dem Zugeben der Cellulase wird die Reaktion unter einer Reaktionstemperatur von 55-65 °C für eine Reaktionszeit von 2 h - 4 h durchgeführt;
die Inaktivierung wird durch Kochen für 25-35 min durchgeführt;
die Zentrifugierung wird unter Bedingungen von 3500-4500 U/min, 15-25 min und Raumtemperatur durchgeführt;
ein Oligosaccharidgehalt in der wässrigen Lösung des Oligosaccharids aus Dendrobium *officinale* beträgt mehr als 80 %;
in Schritt 6) wird die Reinigung durch Durchleiten durch eine G75-Gelsäule durchgeführt; eine Reinheit des Oligosaccharids aus Dendrobium *officinale* beträgt 99,86 %.

8. Verfahren zum Herstellen des Derivats gemäß Anspruch 2, umfassend:
1) Reduzieren einer Fettsäure zu einem Fettalkohol;
2) Glykosylieren des Fettalkohols mit dem Oligosaccharid aus Dendrobium *officinale* gemäß Anspruch 1, um das Derivat zu erhalten.

9. Verfahren gemäß Anspruch 8, wobei in Schritt 1) ein Reaktionsvorgang der Reduzierung einer Fettsäure zu einem Fettalkohol wie folgt ist: Mischen von 9-11 mmol der Fettsäure, 18-22 mmol NaBH₄, 38-42 mmol AlCl₃, 25-29 ml THF und 2-4 ml MeOH für eine Reaktion, und die Reaktion wird unter einer Temperatur von 95-105 °C für eine Zeit von 7,5-8,5 h mit einer Ausbeute von 88-92 % durchgeführt;
in Schritt 2) ist ein Reaktionsvorgang der Glykosylierung wie folgt: Mischen des Alkohols und des Saccharids in einem Molverhältnis von 1 : (8-10) und dann Verwenden von Cetylammoniumbromid als Katalysator zur Katalyse, und die Reaktion wird unter einer Temperatur von 110-115 °C für eine Zeit von 2,5-3,5 h mit einer Ausbeute von 78-82 % durchgeführt.

## Revendications

1. Oligosaccharide de Dendrobium *officinale,* ayant une structure représentée par la formule (I) :

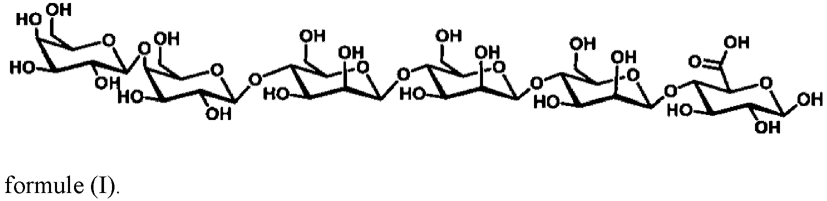

2. Dérivé d'un oligosaccharide de Dendrobium *officinale,* dans lequel le dérivé est généré en faisant réagir le résidu d'acide glucuronique de l'oligosaccharide de Dendrobium *officinale* selon la revendication 1 avec un résidu hydrophobe, et le résidu hydrophobe comprend l'un quelconque parmi un alkyle en C₁ à C₁₈ linéaire ou ramifié, un acide alkylcarboxylique en C₁ à C₁₈, une alkylamine en C₁ à C₁₈, un arylalkyle en C1 à C18 et un alkylamide en C₁ à C₁₈ , ou
le résidu hydrophobe comprend l'un quelconque parmi un benzoyle, un phénylacétyle et un phénylpropionyle.

3. Oligosaccharide de Dendrobium *officinale* selon la revendication 1 ou dérivé selon la revendication 2 pour une utilisation dans la préparation de produits antivieillissement ou immunomodulateurs.

4. Oligosaccharide de Dendrobium *officinale* ou dérivé selon la revendication 3, dans lequel les produits comprennent des médicaments, des aliments, des formulations et des cosmétiques.

5. Oligosaccharide de Dendrobium *officinale* ou dérivé selon la revendication 4, dans lequel un type des cosmétiques comprend une crème pour le visage, une émulsion, une lotion de tonte ou une crème de réparation.

6. Procédé pour extraire l'oligosaccharide de Dendrobium *officinale* selon la revendication 1, comprenant :
1) la préparation d'une poudre de Dendrobium officinale : sécher une tige de Dendrobium officinale, puis broyer et tamiser pour obtenir une poudre de Dendrobium *officinale* ;
2) la préparation d'un extrait aqueux de Dendrobium *officinale* : mélanger la poudre de Dendrobium *officinale* avec de l'eau, soumettre à un bain d'eau bouillante pendant une ou plusieurs fois, puis mélanger des extraits aqueux pour obtenir un extrait aqueux de Dendrobium *officinale* ;
3) la préparation d'un extrait aqueux concentré de Dendrobium officinale : centrifuger l'extrait aqueux de Dendrobium officinale, puis concentrer sous pression réduite pour obtenir un extrait aqueux concentré de Dendrobium *officinale* ;
4) la préparation d'un liquide de redissolution : ajouter de l'éthanol à l'extrait aqueux concentré de Dendrobium *officinale*, précipiter, centrifuger puis éliminer un surnageant pour obtenir un précipité ; ajouter de l'eau au précipité obtenu pour redissoudre pour obtenir un liquide redissouvant ;
5) la préparation d'une solution aqueuse de l'oligosaccharide de Dendrobium *officinale* : déprotéiniser le liquide redissouvant, et lyophiliser pour obtenir un polysaccharide brut de Dendrobium *officinale* ; ajouter de l'eau pour dissoudre le polysaccharide brut de Dendrobium *officinale*, puis ajouter de la cellulase pour une réaction, puis inactiver et centrifuger, et éliminer un précipité pour obtenir une solution aqueuse de l'oligosaccharide de Dendrobium *officinale* ;
6) la préparation de l'oligosaccharide de Dendrobium officinale : purifier la solution aqueuse de l'oligosaccharide de Dendrobium officinale pour obtenir l'oligosaccharide de Dendrobium officinale.

7. Procédé selon la revendication 6, dans lequel,
à l'étape 1), une température pour sécher est de 50 à 60 °C ; une teneur massique en humidité de la tige de Dendrobium *officinale* après sécher est inférieure à 6 % ; un nombre de mailles du tamisage est de 20 à 40 mailles ;
à l'étape 2), la poudre de Dendrobium *officinale* et de l'eau sont mélangées dans un rapport masse-volume de 1 : (50 à 80) g/ml ; le bain d'eau bouillante est effectué pendant 2 à 3 fois ; un temps pour chaque bain d'eau bouillante est de 1 à 2 h ;
à l'étape 3), la centrifugation est effectuée dans des conditions de 3000 à 4000 tr/min, de 8 à 12 min et de température ambiante ; la concentration sous pression réduite est effectuée dans des conditions d'un procédé d'évaporation rotative, d'une température de recyclage d'éthanol inférieure à 50 °C et d'une pression de 0,7 Pa à 0,9 Pa ; un rapport masse-volume matière-liquide de l'extrait aqueux concentré de Dendrobium *officinale* est de 1 : (5 à 10) g/ml ;
à l'étape 4), l'addition d'éthanol à l'extrait aqueux concentré de Dendrobium *officinale* et la précipitation sont spécifiquement effectuées comme suit : ajouter de l'éthanol avec une concentration volumique de 95 % à l'extrait aqueux concentré de Dendrobium *officinale* jusqu'à ce qu'une concentration en alcool d'un mélange soit de 70 % à 80 % en pourcentage volumique, puis précipiter pendant une nuit ;
la centrifugation est effectuée dans des conditions de 3500 à 4500 tr/min, de 18 à 22 min et de température ambiante ; un rapport poids-volume du précipité et de l'eau pour redissoudre est de 1 : (4 à 6) g/ml ;
à l'étape 5), la déprotéinisation du liquide de redissoudre est effectuée par un procédé Sevage ;
une température pour lyophiliser est de 45 °C ;
l'addition d'eau pour dissoudre le polysaccharide brut de Dendrobium *officinale* est effectuée comme suit : ajouter de l'eau au polysaccharide brut de Dendrobium *officinale* pour dissoudre jusqu'à 4,5 à 5,5 mg/ml ;
une quantité ajoutée de la cellulase est de 2 % à 4 % d'un poids du polysaccharide brut de Dendrobium *officinale* ;
après ajouter la cellulase, la réaction est effectuée à une température de réaction de 55 à 65 °C pendant un temps de réaction de 2 h à 4 h ;
l'inactivation est effectuée en bouillant pendant 25 à 35 min ;
la centrifugation est effectuée dans des conditions de 3500 à 4500 tr/min, de 15 à 25 min et de température ambiante ;
une teneur en oligosaccharide dans la solution aqueuse de l'oligosaccharide de Dendrobium *officinale* est supérieure à 80 % ;
à l'étape 6), la purification est effectuée en passant à travers une colonne de gel G75 ; une pureté de l'oligosaccharide de Dendrobium *officinale* est de 99,86 %.

8. Procédé pour préparer le dérivé selon la revendication 2, comprenant :
1) réduire un acide gras en un alcool gras ;
2) glycosyler l'alcool gras avec l'oligosaccharide de Dendrobium *officinale* selon la revendication 1 pour obtenir le dérivé.

9. Procédé selon la revendication 8, dans lequel, à l'étape 1), un procédé de réaction de la réduction d'un acide gras en un alcool gras est comme suit : mélanger 9 à 11 mmol de l'acide gras, 18 à 22 mmol de NaBH₄, 38 à 42 mmol d'AlCl₃, 25 à 29 ml de THF et 2 à 4 ml de MeOH pour une réaction, et la réaction est effectuée à une température de 95 à 105 °C pendant un temps de 7,5 à 8,5 h avec un rendement de 88 à 92 % ;
à l'étape 2), un processus de réaction de la glycosylation est comme suit : mélanger l'alcool et le saccharide dans un rapport molaire de 1 : (8 à 10), puis utiliser du bromure de cétylammonium comme catalyseur pour la catalysation, et la réaction est effectuée à une température de 110 à 115 °C pendant un temps de 2,5 à 3,5 h avec un rendement de 78 à 82 %.
